# EUROPEAN PATENT APPLICATION

(11) **EP 3 228 708 A1**
(43) Date of publication of application: **11.10.2017**
(21) Application number: 15865918.5
(22) Date of filing: 02.12.2015
(51) Int. Cl.: C12N 15/09, A01K 67/027

(54) **NON-HUMAN MAMMAL**

(30) Priority: 03.12.2014 JP 2014244988
(71) Applicant: Tokyo Metropolitan Institute of Medical Science, Tokyo 156-8506 (JP)
(72) Inventor: KAMINUMA, Osamu, Tokyo 156-8506 (JP); INOUE, Kimiko, Saitama 351-0198 (JP); KATAYAMA, Kazufumi, Osaka 561-0825 (JP); OGURA, Atsuo, Saitama 351-0198 (JP)
(74) Representative: Vossius & Partner Patentanwälte Rechtsanwälte mbB
(86) International application number: PCT/JP2015/083869
(87) International publication number: WO 2016/088799

(57) **Abstract**

A non-human mammal and an offspring thereof, obtainable by a somatic cell nuclear transfer method using a nucleus of a CD4-positive T cell as a nuclear donor. The non-human mammal of the present invention surely and efficiently shows allergic reactions specific to various antigens that are shown to have associations with an immune allergic disease, such as mites and cedar pollens, so that the non-human mammal can be suitably used as the developmental models of allergic diseases for studies of various diseases by studying or pursuing possibilities of applications to the diseases.

SEQUENCE LISTING

<110>  Tokyo Metropolitan Institute of Medical Science

<120>  A non-human mammal

<130>  15-037-PCTJP

<150>  JP 2014-244988
<151>  2014-12-03

<160>  7

<170>  PatentIn version Ver. 3.5.1

<210> 1
<211> 20
<212> PRT
<213> Dermatophagoides pteronyssinus

<400> 1
Cys His Gly Asp Thr Ile Pro Arg Gly Ile Glu Tyr Ile Gln His Asn
1               5                   10                  15

Gly Val Val Gln
            20

<210> 2
<211> 20
<212> PRT
<213> Dermatophagoides pteronyssinus

<400> 2
Glu Tyr Ile Gln His Asn Gly Val Val Gln Glu Ser Tyr Tyr Arg Tyr
1               5                   10                  15

Val Ala Arg Glu
            20

<210> 3
<211> 20
<212> PRT
<213> Dermatophagoides pteronyssinus

<400> 3
Glu Ser Tyr Tyr Arg Tyr Val Ala Arg Glu Gln Ser Cys Arg Arg Pro
1               5                   10                  15

Asn Ala Gln Arg
            20

<210> 4
<211> 20
<212> PRT
<213> Dermatophagoides pteronyssinus

<400> 4
Arg Arg Pro Asn Ala Gln Arg Phe Gly Ile Ser Asn Tyr Cys Gln Ile
1               5                   10                  15

Tyr Pro Pro Asn
            20



<210> 5
<211> 20
<212> PRT
<213> Dermatophagoides pteronyssinus

<400> 5
Phe Gly Ile Ser Asn Tyr Cys Gln Ile Tyr Pro Pro Asn Val Asn Lys
1               5                   10                  15

Ile Arg Glu Ala
            20



<210> 6
<211> 20
<212> PRT
<213> Dermatophagoides pteronyssinus

<400> 6
Pro Pro Asn Val Asn Lys Ile Arg Glu Ala Leu Ala Gln Thr His Ser
1               5                   10                  15

Ala Ile Ala Val
            20



<210> 7
<211> 20
<212> PRT
<213> Dermatophagoides pteronyssinus

<400> 7
Leu Ala Gln Thr His Ser Ala Ile Ala Val Ile Ile Gly Ile Lys Asp
1               5                   10                  15

Leu Asp Ala Phe
            20

## Description

### TECHNICAL FIELD

The present invention relates to a non-human mammal. More particularly, the present invention relates to a cloned non-human mammal which can be used as an allergy model animal obtainable by a somatic cell nuclear transfer method, and a method for preparing the non-human mammal.

### BACKGROUND ART

Live bodies have been protected from foreign substances primarily by immune responses, and an immune system which is composed of various cells and factors produced thereby. In particular, leukocytes, especially lymphocytes, play a key role. The lymphocytes are classified in two major types, B lymphocytes (which may be hereinafter referred to as B cells) and T lymphocytes (which may be hereinafter referred to as T cells), both of which specifically recognize an antigen and act to protect the live bodies from the antigen.

Peripheral T cells are mostly composed of CD(Cluster of Differentiation)4-positive cells having a CD4 marker and CD8-positive T cells having a CD8 marker. The majority of the CD4-positive T cells are called helper T cells, which are involved in assistance of antibody production and induction of various immune responses, and are differentiated into subsets of Th1, Th2, and Th17 cells, depending upon the kinds of cytokines produced by antigen stimulations. The majority of the CD8-positive T cells are differentiated into cytotoxic T cells showing cytotoxic activity by antigen stimulations.

It is crucial to analyze specific biological reactions against foreign antigens in view of elucidating mechanisms of the development of immune diseases such as allergies caused thereby and of developing the prevention or treatment methods therefor. For example, when a foreign antigen invades into a live body, CD4-positive T cells that specifically recognize the foreign antigen would lead to turn on various biological reaction cascades intended for live body protection. So far, various studies have been carried out in order to clarify the functions and roles of the antigen-specific CD4-positive T cells.

It is possible to proliferate antigen-specific CD4-positive T cells in live bodies by administering the antigen together with a proper adjuvant substance to experiment animals such as mice, and the antigen-specific reactions can be observed in those animals at individual levels and *ex vivo.* Further, a single antigen-specific CD4-positive T cell-originated cells can be obtained by co-culturing CD4-positive T cells that are taken from antigen-sensitized animals with a specific antigen and antigen-presenting cells, followed by a cloning process with limiting dilutions.

It is crucial to analyze reactivities of a single CD4-positive T cell in view of clarifying its differentiation mechanisms, the signal transduction mechanisms via T cell receptor (TCR), and variances among the cells. Although the single CD4-positive T cell responses can be analyzed *in vivo* by transferring an antigen specific-CD4-positive T cell clone that is previously established *in vitro* into normal mice, in that case all of the CD4-positive T cells in live bodies cannot be antigen-specific. Although it is possible to use T cell-deficient mice as a recipient side, all the presently existing T cell-deficient mice show some sorts of immunodeficiencies, thereby making it difficult to construct an appropriate experimental system in view of analyzing the immune mechanisms mediated by transferred CD4-positive T cells.

In the recent years, DO 11.10, OT-II, and OVA 23-3 mice, and transgenic (Tg) mice transduced with antigen-specific TCR gene disclosed in Patent Publication 1 and Non-Patent Publication 1 have been developed. DO 11.10 and OT-II mice have been frequently used as experimental animals which can solve the problems mentioned above. With the above TCR-Tg mice, CD4-positive T cells having a single TCR can be easily prepared *in vitro,* and the antigen-specific reaction can be easily induced also *in vivo.*

On the other hand, with the advancement of somatic cell cloning techniques, it is possible to generate cloned mice from cells originated from various organs. As those for the origins of a T cell, first, mice derived from T cells of which antigen specificity is unknown have been generated in the year 2002 (see, Non-Patent Publication 2), and since then, cloned mice derived from antigen-specific NKT cells (see, Patent Publication 2), and cloned mice derived from antigen-specific CD8-positive T cells have been generated (see, Patent Publication 3).

### PRIOR ART REFERENCES

### PATENT PUBLICATIONS

Patent Publication 1: WO 2010/107400
Patent Publication 2: WO 2006/018998
Patent Publication 3: WO 2010/003103

### NON-PATENT PUBLICATIONS

Non-Patent Publication 1: E. R. Jarman, K. A. L. Tan, J. R. Lamb. Transgenic mice expressing the T cell antigen receptor specific for an immunodominant epitope of a major allergen of house dust mite develop an asthmatic phenotype on exposure of the airways to allergen. Clin Exp Allergy 2005; 35:960-969.
Non-Patent Publication 2: Hochedlinger K, Jaenisch R. Monoclonal mice generated by nuclear transfer from mature B and T donor cells. Nature 2002; 415:1035-8.

### SUMMARY OF THE INVENTION

### PROBLEMS TO BE SOLVED BY THE INVENTION

However, cloned mice from antigen-specific CD4-positive T cells have not yet been established.

An object of the present invention is to establish the technique of generating a cloned animal from an antigen-specific CD4-positive T cell, and to provide a non-human mammal reactive to various antigens such as mites and cedar pollens, that are suggested to be associated with immunological and allergic diseases.

### MEANS TO SOLVE THE PROBLEMS

The present invention relates to the following [1] to [22]:
[1] A non-human mammal and an offspring thereof, obtainable by a somatic cell nuclear transfer method using a nucleus of a CD4-positive T cell as a nuclear donor.
[2] The non-human mammal and an offspring thereof according to the above [1], wherein the CD4-positive T cell is a CD4-positive T cell at G₀ phase and/or G₁ phase.
[3] The non-human mammal and an offspring thereof according to the above [1] or [2], wherein a gene region for a T cell receptor is rearranged as a T cell receptor specific to a mite antigen.
[4] The non-human mammal and an offspring thereof according to the above [3], wherein the mite antigen is a mite body or excrement of *Dermatophagoides farinae or Dermatophagoides pteronyssinus,* or an extract, a recombinant protein or a synthetic peptide thereof.
[5] The non-human mammal and an offspring thereof according to the above [1] or [2], wherein a gene region for a T cell receptor is rearranged as a T cell receptor specific to an egg albumin.
[6] A method for preparing a non-human mammal including performing a somatic cell nuclear transfer using a nucleus of a CD4-positive T cell as a nuclear donor.
[7] The method according to the above [6], wherein the CD4-positive T cell is a CD4-positive T cell obtainable by adding IL-2 thereto, culturing the mixture, thereafter removing IL-2, and further culturing the cultured mixture.
[8] The method according to the above [6] or [7], wherein a gene region for a T cell receptor is rearranged as a T cell receptor specific to a mite antigen.
[9] The method according to the above [8], wherein the mite antigen is a mite body or excrement of *Dermatophagoides farinae or Dermatophagoides pteronyssinus,* or an extract, a recombinant protein or a synthetic peptide thereof.
[10] The method according to the above [6] or [7], wherein a gene region for a T cell receptor is rearranged as a T cell receptor specific to an egg albumin.
[11] A non-human mammal wherein a gene region for a T cell receptor is rearranged as a T cell receptor specific to a mite antigen or an egg albumin, and wherein a single T cell receptor derived from the gene region is expressed in an individual.
[12] The non-human mammal according to the above [11], wherein the mite antigen is a mite body or excrement of *Dermatophagoides farinae or Dermatophagoides pteronyssinus*, or an extract, a recombinant protein or a synthetic peptide thereof.
[13] A non-human mammal wherein a gene region for a T cell receptor is rearranged as a T cell receptor specific to an egg albumin, and wherein a single T cell receptor derived from the gene region is expressed in an individual.
[14] An allergic model mouse obtainable by a somatic cell nuclear transfer method using a nucleus of a CD4-positive T cell as a nuclear donor.
[15] The allergic model mouse according to the above [14], wherein the CD4-positive T cell is a CD4-positive T cell at G₀ phase and/or G₁ phase.
[16] The allergic model mouse according to the above [14] or [15], wherein a gene region for a T cell receptor is rearranged as a T cell receptor specific to a mite antigen.
[17] The allergic model mouse according to the above [16], wherein the mite antigen is a mite body or excrement of *Dermatophagoides farinae or Dermatophagoides pteronyssinus,* or an extract, a recombinant protein or a synthetic peptide thereof.
[18] The allergic model mouse according to the above [14] or [15], wherein a gene region for a T cell receptor is rearranged as a T cell receptor specific to an egg albumin.
[19] A method of using a non-human mammal and an offspring thereof as defined in any one of the above [1] to [5] and [11] to [13] as an allergic model mouse.
[20] A method for generating an allergic model mouse including subjecting a non-human mammal and an offspring thereof as defined in any one of the above [1] to [5] and [11] to [13] to antigen sensitization.
[21] An allergic model mouse obtainable by a method as defined in the above [20].
[22] A cell, a tissue, an organ or a product obtainable from a non-human mammal and an offspring thereof as defined in any one of the above [1] to [5] and [11] to [13].

### EFFECTS OF THE INVENTION

The non-human mammal of the present invention exhibits some excellent effects of surely and efficiently showing allergic reactions specific to a mite antigen, a cedar pollen, an egg albumin or the like. In addition, the developmental mechanisms of allergic diseases caused by specific biological reactions against a mite antigen, a cedar pollen, an egg albumin or the like are elucidated by using the non-human mammal of the present invention as an allergic model animal, thereby making it possible to contribute to the development of methods of prevention and treatment thereof.

### BRIEF DESCRIPTION OF THE DRAWINGS

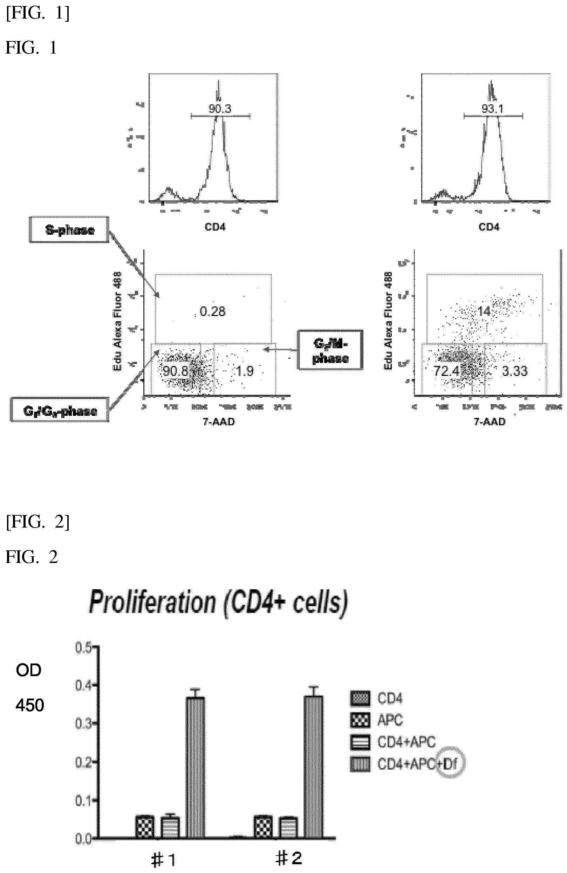

[FIG. 1] FIG. 1 is diagrams showing one example of the results of the preparation of antigen-specific CD4-positive T cells. With respect to the CD4-positive T cells of sensitized mice, the results of a case where addition of IL-2 is controlled (left diagrams), and the results of a case where IL-2 is continued to be added (right diagrams) are shown. Here, the upper diagrams show purity of the CD4-positive T cells after the preparation, and the lower diagrams show cell cycle patterns.
[FIG. 2] FIG. 2 is a diagram showing the results of analyzing antigen-specific proliferative responses in peripheral blood CD4-positive T cells using two cloned mice (Df#1, Df#2) obtained by using Mite Extract-Df (LG5339) as an antigen, and CFA as an adjuvant. In the figure, CD4 is a case where CD4-positive T cells were singly cultured; APC is a case where an antigen-presenting cell obtained by irradiating splenocytes of normal mice with X-ray was singly cultured; CD4 + APC is a case where CD4 and APC were mixed and cultured; and CD4 + APC + Df is a case where CD4, APC, and a mite antigen were mixed and cultured.

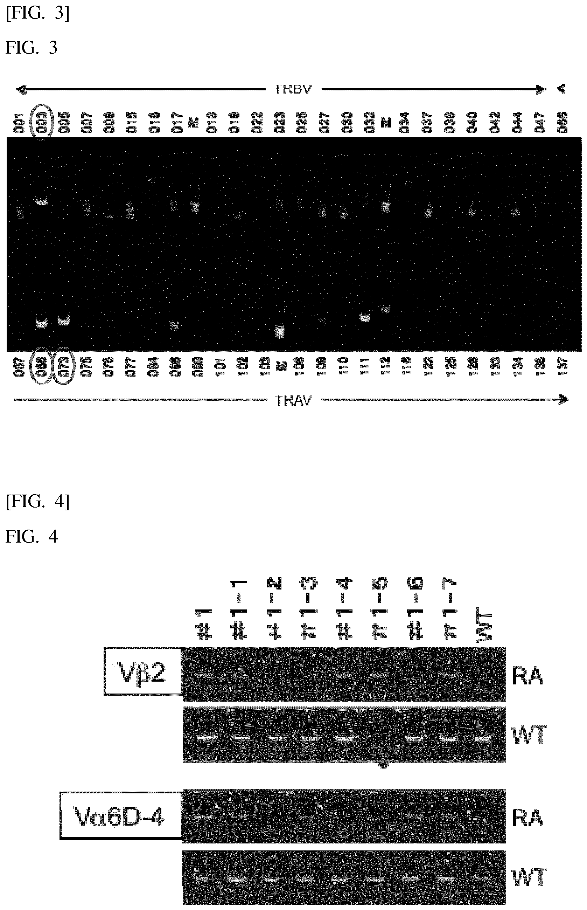

[FIG. 3] FIG. 3 is a view showing results of cloning of TCRα chains (TRAV)/TCRβ chains (TRBV) in peripheral blood CD4-positive T cells of cloned mice (Df#1). The numerical values in the figure show the number of primer sets that are originally assigned, and M is a marker.
[FIG. 4] FIG. 4 is view showing the results of TCR genotyping of cloned mice and F1 mice thereof. In the figure, parental mice (#1), F1 mice (#1-1 to -7), and wild-type mice (WT) are shown.

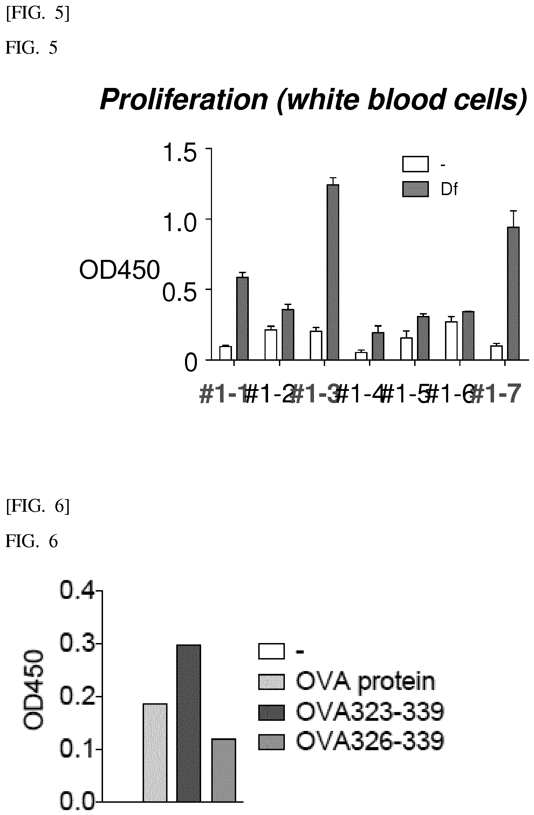

[FIG. 5] FIG. 5 is a diagram showing the results of analyzing antigen-specific proliferative responses in F1 mice of the cloned mice. In the figure, "-" column is a case where a nonspecific antigen was reacted, and "Df" column is a case where a specific antigen was reacted.
[FIG. 6] FIG. 6 is a diagram showing the results of analyzing antigen-specific proliferative responses in peripheral white blood cells using cloned mice (OVA#6) obtained by using a partial peptide OVA 326-339 as an antigen. In the figure, - is a case where CD4-positive T cells were singly cultured; OVA protein is a case where OVA protein and APC were mixed and cultured; OVA 326-339 is a case where a partial peptide OVA 326-339 and APC were mixed and cultured; and OVA 323-339 is a case where a partial peptide OVA 323-339 and APC were mixed and cultured.

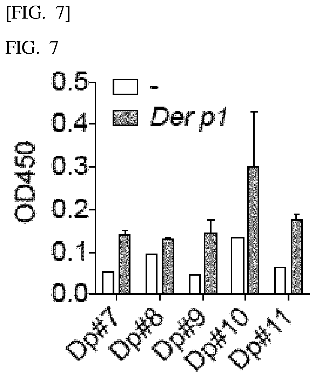

[FIG. 7] FIG. 7 is a diagram showing the results of analyzing antigen-specific proliferative responses in peripheral white blood cells using cloned mice (Dp#7 to #11) obtained by using Der p1 (RP-DP1D-1) as an antigen, and CFA as an adjuvant. In the figure, - is a case where CD4-positive T cells were singly cultured, and *Der p1* is a case where *Der p1* and APC were mixed and cultured.

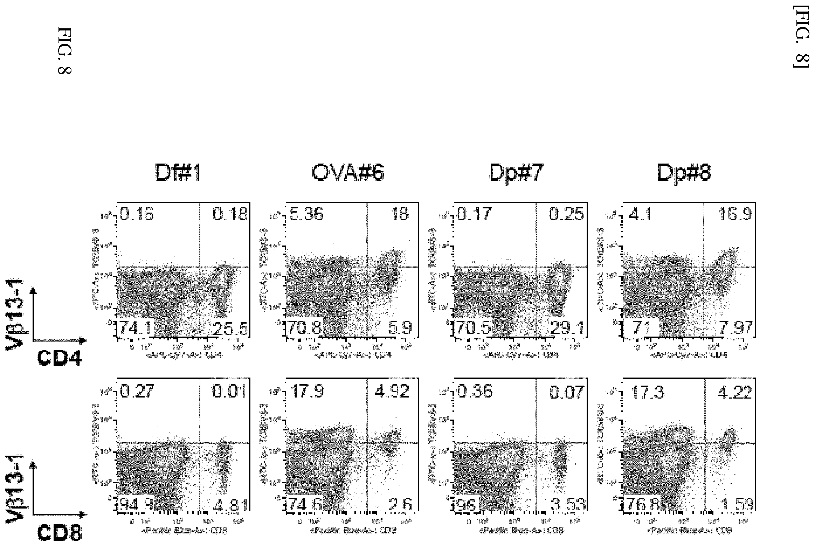

[FIG. 8] FIG. 8 is diagrams showing the results of analyzing expression of rearranged TCR in T cells of cloned mice according to flow cytometry. The upper row is the results in CD4 cells, and the lower row is the results in CD8 cells.

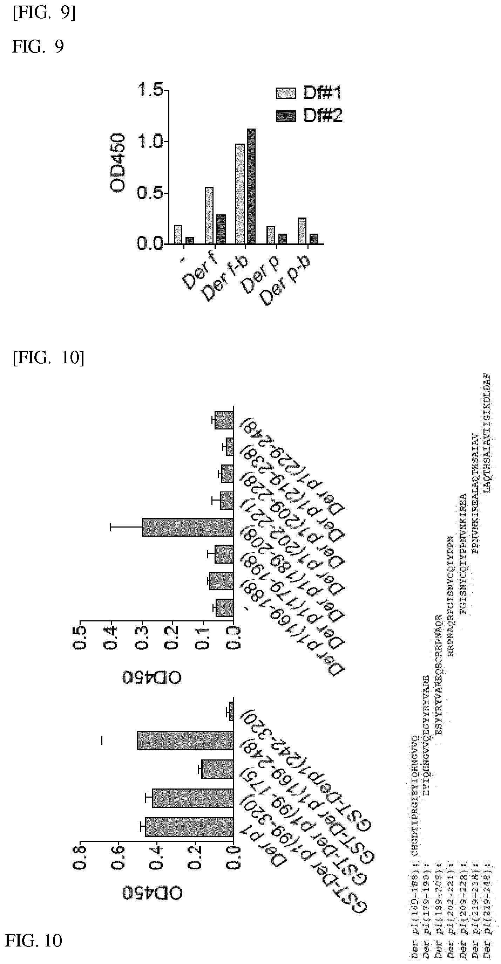

[FIG. 9] FIG. 9 is a diagram showing the results of analyzing antigen-specific proliferative responses in cloned mice (Df#1, Df#2).
[FIG. 10] FIG. 10 is diagrams showing the results of analyzing antigen-specific proliferative responses in cloned mice (Dp#7). The left panel is the results of examining a whole protein and partial peptides thereof, and the right panel is the results of examining epitopes for the peptide regions showing reactivities in the left panel. Here, the details of the partial peptides used are shown at the bottom of the figure.

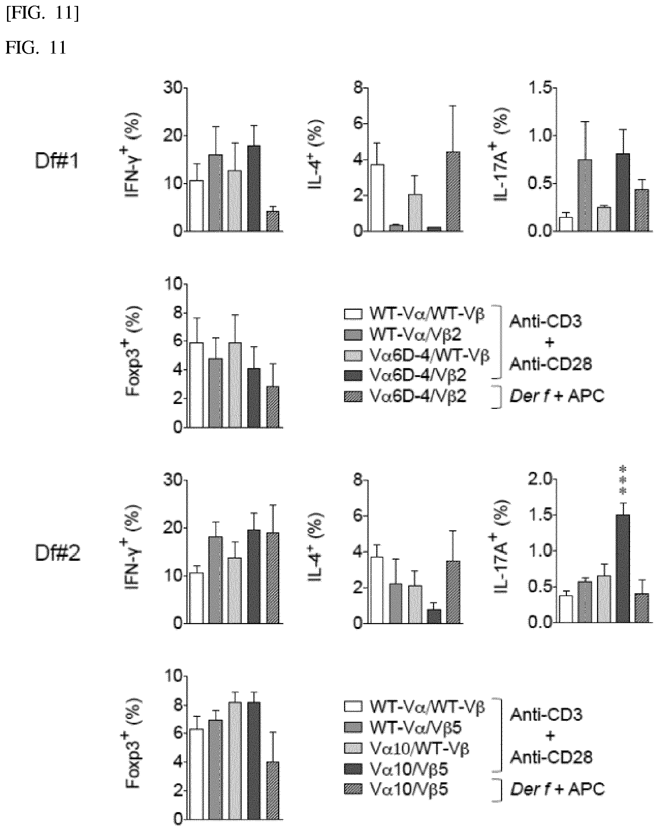

[FIG. 11] FIG. 11 is diagrams showing the results of analyzing differentiation ability of CD4-positive T cells of cloned mice (Dp#1, Dp#2) according to flow cytometry. The upper half of the diagrams are the results for Dp#1 mice, and the lower half of the diagrams are the results for Dp#2 mice. Here, "***" means P < 0.001 according to Dunnett's method.

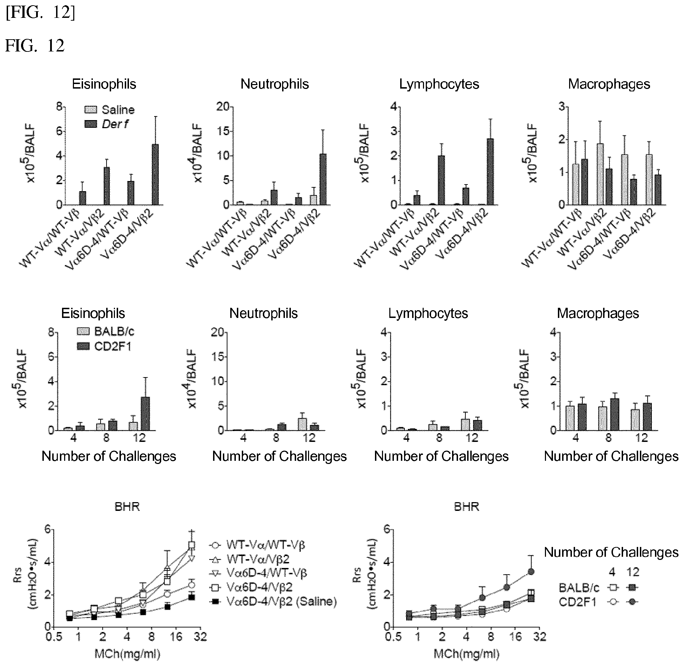

[FIG. 12] FIG. 12 is the results of studying the development of bronchial asthma-like airway inflammation of cloned mice (Dp#1). In the figure, the upper rows shows the results for inflammatory cell counts in cloned mice having rearranged TCR, and the lower row shows the results of inducing bronchial asthma-like airway inflammation in normal mice.

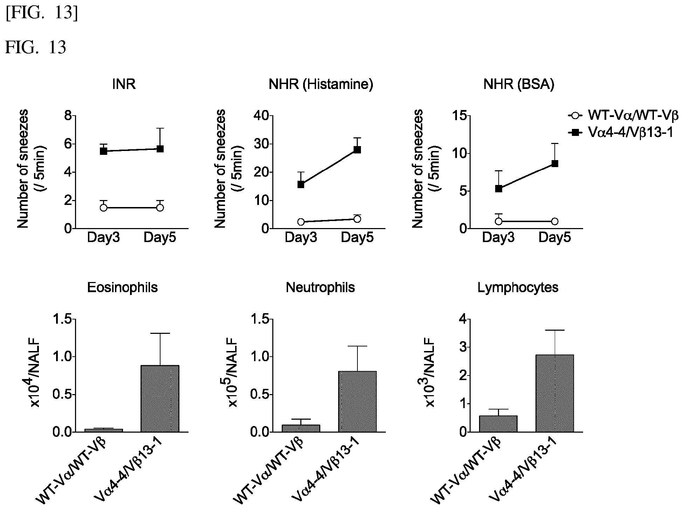

[FIG. 13] FIG. 13 is diagrams showing the results of studying the development of antigen-induced nasal reactions in cloned mice (OVA#6). The upper row is diagrams for the results of rhinitis reactions (sneezing reactions), and the lower row is diagrams for nasal responses (inflammatory cell counts).

### MODES FOR CARRYING OUT THE INVENTION

The non-human mammal of the present invention has a great feature in that the non-human mammal is obtainable by a somatic cell nuclear transfer method (a somatic cell cloning method) using a nucleus of an antigen-specific CD4-positive T cell as a nuclear donor. Here, in the present specification, the non-human mammal of the present invention may be described as a cloned animal or cloned mammal of the present invention.

A transgenic (Tg) animal is obtainable by transferring an artificially recombinant DNA-transduced fertilized egg. For example, the expression of T cell receptor (TCR) in CD4-positive T cells in all presently existing MHC-class II-restricted TCR-Tg mice (hereinafter represented as TCR-Tg mice) are regulated by an extrinsic promoter, so that the expression levels thereof and the timing of expression are different from endogenous TCR. In addition, since the reactivities of T cells caused by TCR stimulations are known to be different according to affinity to an antigen or expression levels of the TCR, it is not clear that the reactions of CD4-positive T cells in the TCR-Tg mice completely reflect the reactions of antigen-specific CD4-positive T cells naturally developed in live bodies. In fact, it is said that T cells in Balb/c mice are more likely to cause a so-called Th2 type response by antigen sensitization; it has been reported that Th17/Th1-favored reactions are induced when antigen-specific reactions are observed in individual levels with DO 11.10 mice as the background (see, Lemaire MM, Dumoutier L, Warnier G, Uyttenhove C, Van Snick J, de Heusch M, Stevens M, Renauld JC. Dual TCR expression biases lung inflammation in DO 11.10 transgenic mice and promotes neutrophilia via microbiota-induced Th17 differentiation. J Immunol 2011; 187:3530-7.). The cloned animal obtainable in the present invention has a CD4-positive T cell which expresses an antigen-specific TCR, and the cloned animal is a somatic cell cloned animal in which a nucleus obtained from an antigen-specific CD4-positive T cell in a nearly live body state is used as a nuclear donor, and in which the same gene information as the CD4-positive T cell of the original nucleus provider is handed down. Therefore, it is assumed that an unnatural reaction which is apprehensive for TCR-Tg mice does not occur, but an antigen-specific reaction perfectly reflecting the reaction of an antigen-specific CD4-positive T cell that is naturally developed in a live body can be observed. Although biological cascades caused by mite antigens, for example, are seen as the multiple phenotypes of numerous specific reactions to epitopes in the mite antigen proteins, animals that are only specifically reactive to each epitope can be obtained according to the techniques in the present invention, so that the characteristics of each of epitopes and their reactive CD4-positive T cells can be analyzed in detail. In order to perform similar analysis utilizing TCR-Tg mice, it is necessary to perform TCR cloning, then generate gene-transduced mice, and check for the transduced gene in each of individuals obtained. According to the technique of generating an antigen-specific CD4-positive T cell cloned animal provided by the present invention, it is possible to omit these steps, so that a more efficient method for analyzing antigen-specific reactions can be provided.

The non-human mammal as used herein is, but not particularly limited to, a mammal other than human, and preferably rodents such as mice, rats, guinea pigs, and rabbits are used, among which mice are preferred. The mice used are not particularly limited, and known mice can be used, and in the present invention, CD2F1 (BALB/c x DBA2) mice are preferably used, from the viewpoint that the generation efficiency of transnuclear mice is higher in F1 mice than the inbred mice, that the histocompatibility antigen haplotypes in BALB/c and DBA2 mice are almost completely equivalent, and the like.

The CD4-positive T cell used in the present invention is, but not particularly limited to, an antigen-specific T cell, and the antigen to be subject includes, for example, indoor dust and insect antigens such as mites, pollen antigens such as cedar pollens, Japanese cypress pollens, and rice pollens, edible antigens such as soybeans and eggs, and the like. The present invention will be described hereinbelow taking an embodiment where a mite antigen-specific T cell is used as an example, without intending to limit the present invention thereto.

The mite antigen in the present invention is not particularly limited, and the species of mites are also not limited. Examples include *Dermatophagoides farinae* (Df), *Dermatophagoides pteronyssinus* (Dp), and the like, and a mite body or excrement thereof, an extract, a recombinant protein, a synthetic peptide prepared therefrom according to known methods, and commercially available products thereof can be suitably used. Commercially available products include manufactured articles such as Mite Extract-Df, Mite-Df Feces AG, Mite Extract-Dp, Mite-Dp Feces AG (hereinabove manufactured by SLS), Der f1, Der f2 (hereinabove manufactured by ASAHI FOOD & HEALTHCARE CO., LTD.), Der fII (manufactured by SHIBAYAGI Co., Ltd.), *Dermatophagoides farinae* mite body and *Dermatophagoides pteronyssinus* mite body (hereinabove manufactured by Biostir Inc.). These can be used alone or in a combination of two or more kinds.

The mite antigen-specific T cell used in the present invention is a mite allergen-specific CD4-positive T cell obtained usually by sensitization with the above mite allergen. The antigen sensitization can be performed according to a known method, and an antigen can also be sensitized together with a known adjuvant. In addition, in order for obtaining a sufficient number of CD4-positive T cells for performing nuclear transfer, T cells may be obtained by culturing the cells in the copresence of a known factor having a proliferation-inducing ability of CD4-positive T cells and a mite allergen. The proliferation (growth)-inducing factor includes IL-2 and IL-4, and the like. The T cells obtained can be properly screened and harvested by, for example, performing FACS analysis or a known co-culture. Here, the culture conditions as used herein can be appropriately set depending upon the kinds of T cells. In the present invention, it is preferable that the T cell is cells at G₀ phase and/or G₁ phase, from the viewpoint of dramatically lowering the generation efficiency of the cloned animal in a case where cells in a progressive cell cycle are used. The cells of the above state can be collected, for example, from a cell population of the antigen specific CD4-positive T cells synchronous to G₀ phase and/or G₁ phase, which is obtained by adding a growth-inducing factor thereto, culturing the cells, removing the factor therefrom, and further culturing the cells.

In addition, in another embodiment, in order for obtaining a cell population of antigen-specific CD4-positive T cells synchronous to G₀ phase and/or G₁ phase, a pretreatment for reducing the proliferation of antigen-nonspecific T cells may be performed. More concretely, the proliferation of antigen-nonspecific T cells can be reduced by not adding a growth inducing factor upon co-culturing a cell population of CD4-positive T cells collected from an antigen-sensitized mammal together with splenic cells pretreated to lose the proliferation ability and a specific antigen. The cell population of antigen-specific CD4-positive T cells synchronous to G₀ phase and/or G₁ phase, can be obtained at a high efficiency by adding a growth inducing factor to the cell population in the above state, culturing the cells, thereafter removing the growth inducing factor therefrom, and further culturing the cells. The culture period of these cells may be, but not particularly limited to, a culture period of several days, and preferably a culture period of from 3 to 4 days. In order for synchronizing T cells to G₀ phase and/or G₁ phase, it is preferable that the proliferation inducing factor is IL-2. Here, it is preferable that the kinds of the T cells are homogenous to that of the non-human mammal produced.

The mite antigen-specific CD4-positive T cell thus obtained expresses TCR which is capable of binding to a mite antigen epitope presented on the cell surface with MHC-class II, in addition to having a CD(Cluster of Differentiation)4 marker on a cell membrane. The TCR capable of binding to the mite antigen is usually composed of heterodimers of α chains or β chains, and recognizes an antigen on the antigen-presenting cell, and any of the α chains and the β chains are arranged by variable regions (V regions) and constant regions (C regions), and rearranged (restructured) upon the reaction to an antigen. More particularly, first, upon the reaction to an antigen, VDJ rearrangement of the β chains takes place, and subsequently VJ rearrangement of the α chains takes place. Since a wild animal has an opportunity of being exposed to various antigens in nature, each of T cells existing in the body usually expresses different TCRs corresponding to numerous antigen epitopes, and each of single mite allergen-specific TCRs composed of heterodimers of α chains or β chains that are ordinarily rearranged owned by each of the cloned mice in the present invention has a feature of already being expressed in nearly all of T cells in the body before the reaction with the antigen. Due to the genetic construction of TCR, it is possible to trace the origin of the donor cells in the cloned animal obtained.

The nuclear transfer is performed using the above mite antigen-specific CD4-positive T cell. The method for the nuclear transfer is, but not particularly limited to, a method using a nucleus of a mite antigen-specific CD4-positive T cell as a nuclear donor. For example, a method described in Nature 1998; 394:369-74 can be referred.

Concretely, a nucleus of a mite antigen-specific CD4-positive T cell may be replaced with a nucleus of an unfertilized ovum derived from a mammal of the same species as the cell. For example, a nucleus of unfertilized oocytes and the surrounding cytoplasm are subjected to suction-enucleation with a fine pipette to the oocytes, and the nucleus extracted from the above mite allergen-specific CD4-positive T cell is introduced thereinto similarly with the pipette. In addition, nuclear transfer may be performed by introducing a mite allergen-specific CD4-positive T cell itself containing a nuclear donor in place of the nuclear donor to allow cell fusion. Upon the enucleation, a treatment of disappearance of cytoskeleton may be previously performed, or the same treatment may be carried out after the nuclear transfer. The disappearance of the cytoskeleton can be carried out with cytochalasin.

The oocytes to be subjected to nuclear transfer are, but not particularly limited to, oocytes derived from a mammal of the same species as the cell of the nuclear donor. For example, the oocytes can be obtained according to a known superovulation treatment. In addition, the oocytes may or may not be activated, and an activated state is preferred. The activation which may be performed either before or after the nuclear transfer can be performed by stimulations by electric treatment methods or drug treatment methods. Concretely, the activation can be performed by increasing a divalent cation concentration in the oocytes, lowering a degree of phosphorylation of the cellular protein in the oocytes, or the like. For example, the activation is performed by introducing a divalent cation (e.g., magnesium, strontium, barium, or calcium) into a cytoplasm of the oocytes in the form of ionophore. Other methods for increasing a divalent cation concentration include methods of electric shocks, ethanol treatment, and cage chelating agent treatment. In addition, other methods for lowering a degree of phosphorylation include, for example, a method of adding a kinase inhibitor (e.g., serine-threonine kinase inhibitors such as 6-dimethylaminopurine, 2-aminopurine, and sphingosine). Alternatively, a phosphatase can be introduced into the oocytes to inhibit oxidation.

The cell in which nuclear transfer is performed is cultured until a rearranged embryo is formed, and then transferred to the host mammal to allow development of the rearranged embryo in the host mammal, whereby a cloned animal can be obtained. The term "rearranged embryo" as used herein means an embryo rearranged with the nuclear donor and the cytoplasmic component derived from the oocytes.

The transfer of the rearranged embryo to a host animal can be performed according to a known method in the art. Concretely, for example, the rearranged embryo that is cultured to a 2 to 8 cell phase can be transferred. The culture conditions of the rearranged embryo can be appropriately set depending upon the oocytes, and the rearranged embryo may be cultured together with a feeder cell according to a known method.

The host mammal is, but not particularly limited to, a mammal in which a rearranged embryo can be developed, and the host mammal is preferably a mammal derived from the same species as the collected oocytes, more preferably a pseudopregnant female mammal. For example, in case of mice, pseudopregnant female mice can be obtained by mating female mice of normal menstrual cycle with male mice that are castrated by vasoligation or the like. The above rearranged embryo (cloned embryo) is transferred to the pseudopregnant mice within the uterus, especially transferred within a uterine tube to allow pregnancy and delivery of birth, whereby a cloned animal can be generated. In order for causing implantation and pregnancy of the cloned embryo more surely, it is preferable that tentative parents which are pseudopregnant mice are selected from female mice group of the identical menstrual cycle as the female mice from which the oocytes are collected.

In addition, a cloned animal derived from CD4-positive T cells specific to an antigen other than the mite antigen can also be obtained in the same manner as above. For example, in a case where an egg is used for an antigen, an egg albumin (OVA) and partial peptides derived from the OVA, and the like can be suitably used. These may be synthesized products or commercially available products, which can be used alone or in a combination of two or more kinds. In addition, the length of the partial peptide includes, but not particularly limited to, for example, OVA 326-339, OVA 323-339, and the like. Here, it is preferable that the species of the egg is homogeneous to the non-human mammal produced.

Thus, the cloned animal of the present invention is obtained. In the cloned animal obtained, mite antigen-specific CD4-positive T cells proliferate when sensitized with a mite antigen and induce immune responses, and OVA antigen-specific T cells proliferate when sensitized with OVA antigen and induce immune responses. Here, the origin of the cloned animal can be confirmed by examining the genetic information of TCR in the CD4-positive T cells. Since the cloned animal obtained as described above has a histocompatible antigen identical to the nuclear donor cells, when a tissue or an organ of the cloned animal obtained is transferred to a mammal which is a provider of the nuclear donor, the cloned mammal tissues or organs would be in the state of immunological tolerance without being recognized as non-self, so that immunorejection reactions do not take place.

In addition, an offspring thereof can be obtained using the cloned animal of the present invention. Concretely, the offspring can be obtained by mating the cloned animal developed from the rearranged embryo with a second mammal. As a second mammal, a wild-type animal which is homogeneous to the cloned animal, or an animal or offspring thereof or the like developed from the cloned embryo in the same manner can be used. In the present invention, "offspring" embraces those originated from the cloned animal of the present invention, such as child generation (F1) of which parent is the cloned animal of the present invention, child generation (F2) of which parent is F1, and child generation (F3) of which parent is F2.

In the offspring of the cloned animal of the present invention, a gene region of T cell receptor is rearranged as, for example, a mite antigen-specific T cell receptor or an OVA-specific T cell receptor, and a T cell receptor of a single species derived from the gene region, and preferably only the T cell receptor, is expressed in an individual, so that the offspring has the identical mite antigen-specific TCR or OVA-specific TCR to the cells from which the nuclear donor is collected. Also, the T cell having TCR shows an normal differentiation ability to antigen stimuli. Therefore, the offspring of the cloned animal of the present invention can greatly contribute to studies of diseases and/or pathologies caused by mite antigens or OVA, for example, elucidation of the developmental mechanisms. For example, in order for reproducing diseases and/or pathologies caused by mite antigens or OVA usually using experimental animals, it is necessary that animals are sensitized with a mite antigen or OVA, and thereafter induced with a homogenous antigen according to a known method; however, when the offspring of the cloned animal of the present invention is used, the preparation steps would not be necessitated, or would be remarkably shortened or simplified.

The diseases and/or pathologies caused by mite antigens include allergic diseases, and concrete examples include bronchial asthma, atopic dermatitis, allergic rhinitis, and allergic conjunctivitis.

The diseases and/or pathologies caused by OVA include allergic diseases, and concrete examples include food allergies that induce respiratory system symptoms such as urticarial and coughing, and digestive system symptoms such as diarrhea, stomach ache, and vomit, and bronchial asthma, and atopic dermatitis.

The present invention also provides a method for preparing a non-human mammal including performing a somatic cell nuclear transfer using a nucleus of a mite antigen-specific T cell or an OVA-specific T cell as a nuclear donor.

As the mite antigen-specific T cell, those described in the section of the non-human mammal of the present invention can be used, and the preparation thereof can be performed accordingly. In addition, a method of expressing a mite antigen-specific TCR in T cell and a method for somatic cell nuclear transfer can also be performed in the same manner as the procedures described in the section of the non-human mammal of the present invention. The same applies to the OVA-specific T cell.

In addition, since the non-human mammal of the present invention induces immune responses by antigen sensitization, the non-human mammal can be used as an experimental model. In other words, the present invention also provides an allergic model mouse made from a non-human mammal of the present invention and an offspring thereof. The allergic model mouse can be obtained by sensitizing a non-human mammal of the present invention and an offspring thereof with an antigen corresponding to CD4-positive T cell used in the production thereof. Concretely, for example, model mice showing pathologies of bronchial asthma or allergic rhinitis, atopic dermatitis, allergic conjunctivitis, or a digestive tract allergy can be produced by sensitizing a non-human mammal with a mite antigen or OVA or the like. By using the experimental models showing the pathologies, the analysis of pathologies or the developmental mechanisms are elucidated, and pharmacological efficacy evaluation of a candidate compound for prevention or treatment thereof can be made.

### EXAMPLES

The present invention will be described hereinbelow on the basis of Examples, and Examples are intended to illustrate for better understanding of the present invention, without intending to limit the scope of the present invention to these Examples.

Example 1 (Preparation of Antigen-Specific CD4-Positive T Cells) Mite allergens (*Dermatophagoides farinae* (Df) and *Dermatophagoides pteronyssinus* (Dp)) were mixed with aluminum hydroxide (Alum) or Complete Freund's adjuvant (CFA) in a combination of Table 1, and CD2F1 mice (10-week old, male) were injected with the mixture at tail base. The CD2F1 mice were F1 of BALB/c and DBA2.

One week after the antigen sensitization, inguinal lymph node was excised, and suspended in a culture medium containing fetal bovine sera to prepare the cells. The CD4-positive T cells were concentrated with a magnetic sorting system using CD4 antibody beads, and thereafter the culture of concentrated cells was started together with splenocytes prepared from the identical mice, of which proliferation ability was made deficient by X-ray irradiation, and specific antigens. The cells were cultured without adding IL-2 for 3 to 4 days from the beginning of culture, in order to reduce the proliferation of the antigen-nonspecific T cells, thereafter IL-2 was added thereto, and the cells were cultured for additional 3 to 4 days. By the additional culture, only antigen-reactive CD4-positive T cells were sufficiently proliferated, the cells were then collected, washed, resuspended in a culture medium from which IL-2 was again removed, and the cells were cultured for 3 to 4 days. As a comparative subject, cells that were cultured while allowing to keep IL-2 unremoved during the culture period were used, and the cycle patterns of the cells obtained were examined with Click-IT Edu Flow Cytometry Assay Kit (manufactured by Invitrogen). One example of the results is shown in FIG. 1.

From FIG. 1, as compared to a case of allowing to keep IL-2 unremoved during the culture period, clearly many cells were succeeded in converging in the G₁/G₀ phase in a case where the cells were cultured under the conditions of controlling the addition of IL-2.

### Example 2 (Generation of Transnuclear Mice)

Transnuclear mice were generated using the CD4-positive T cells obtained according to Example 1 as a nuclear donor cell. The embryos were generated as prescribed in the methods described in Nature 1998; 394:369-74, and Biology of Reproduction 2012; 86:1-6, and as the culture medium of the embryos, a KSOM medium as prescribed in a method described in Lawitts J.A., and Biggers J.D. 1993, Culture of preimplantation embryos, Methods Enzymol. 225:153-164 was used. Concretely, ova obtained by superovulation of mature CD2F1B6D2F1 female were enucleated in the presence of 5 µg/mL cytochalasin B, and the nuclear donor cell was injected thereto with a piezo-drive micromanupilator. After the injection, the cells were cultured under the environmental conditions of 37.5°C and 6% CO₂ for 1 to 2 hours, and thereafter activated with a KSOM medium in the presence of 50 nM trichostatin A, 5 µM Latrunculin A, and 3 mM strontium. Subsequently, the cells were cultured for 7 hours in the presence of Latrunculin A, and thereafter cultured in an ordinary KSOM medium. In the next day, a 2-cell phase embryo was transferred to a uterine tube of a pseudopregnant ICR female previously mated with a male of which seminal duct was vasoligated, and 19 days later abdominally sectioned to acquire a newborn offspring. The detailed generation efficiencies were as listed in Table 1.

[Table 1]

**Table 1**

| Immune Antigen | Adjuvant | No. of Embryos Cultured | No. of Embryos Developed to Phase 2 Embryonic Cell Phase (%) (% Based on the No. of Embryos Cultured) | No. of Embryos Fragmented | No. of Embryos Transferred | No. of Embryos Implanted (%) (% Based on No. of Embryos Transferred) | Fetus (%/No. of Fetuses) (% Based on No. of Embryos Transferred) | No. of Live Fetuses |
|---|---|---|---|---|---|---|---|---|
| Mite Extract-Df (LG5339) | Alum | 100 | 48 (96.0) | 50 | 48 | 27 (56.3) | 1 (2.1) | 0 |
| Mite Extract-Df (LG5339) | CFA | 241 | 158 (82.3) | 49 | 158 | 66 (41.8) | 4 (2.5) | 2 |
| Df peptide mix (ME1308) | CFA | 140 | 105 (92.1) | 26 | 91 | 31 (34.1) | 0 (0.0) | 0 |
| Df peptide mix (ME 1300) | CFA | 192 | 139 (93.3) | 43 | 138 | 54 (39.1) | 1 (0.7) | 0 |
| Mite Extract-Df (LG5339) | Alum | 91 | 35 (97.2) | 55 | 35 | 23 (65.7) | 1 (2.9) | 1 |
| Mite Extract-Df (LG5339) | Alum | 35 | 14 (93.3) | 20 | 14 | 8 (57.1) | 2 (14.3) | 2 |
| Derp1 (RP-DP1D-1) | CFA | 148 | 125 (92.6) | 13 | 125 | 66 (52.8) | 8 (6.4) | 5 |

### Example 3 (Antigen Reactivities 1 in CD4-Positive T Cells of Cloned Mice)

White blood cells or CD4-positive T cells were prepared from peripheral blood of the Df-specific CD4-positive T cell cloned mice established in Example 2, and the cells were cultured together with X-ray irradiated splenocytes of normal mice and a specific antigen (Df). On the fourth to fifth day from the beginning of culture, the cell proliferative responses were analyzed using a Cell proliferation reagent (manufactured by Roche Diagnostics). The results are shown in FIG. 2.

From FIG. 2, the antigen-specific proliferative responses were found in the mice sensitized with the Df antigen. It was clarified from this matter that the cloned mice possessing the antigen specificity of the donor cell could be established as intended.

### Example 4 (TCR Cloning of Cloned Mice)

mRNA was prepared from the peripheral blood of the established Df-specific CD4-positive T cell cloned mice, and RT-PCR was performed for each of TCRα chains and TCRβ chains using a library of a set of variant chain-specific primers and constant chain-specific primers. As a result, TCRα chains / TCRβ chains expressed in each of cloned mice could be cloned (see, FIG. 3).

### Example 5 (Genetic Characteristics Relating to Antigen Specificities of Cloned Mice)

The TCR sequence cloned in Example 4 was determined by sequencing, and a primer set for Genotyping for detecting the determined sequence were designed. At the same time, a primer set for detecting wild-type allele were also prepared. The cloned mice were back-crossed with BALB/c mice to generate F1 mice, and each of genomic DNAs was extracted from the F 1 mice as well as the parent mice, and PCR genotyping was performed thereon. The results are shown in FIG. 4.

From FIG. 4, in the parent mice (Df#1), rearrangement of TCRα chains and TCRβ chains and alleles of both of the wild types were detected, so that it could be confirmed that only single-sided alleles were rearranged on the basis of the allelic exclusion mechanism. On the other hand, in F 1 mice (Df#1-1 to -7), wild-type alleles were detected in all the mice and rearranged alleles were handed over to about a half of the offspring based on the Mendel's law. Here, in the F1 mice of Df#1-5, wild type alleles were detected by conducting a retest (the results not shown).

Furthermore, white blood cells were prepared from peripheral blood of the F1 mice (Df#1-1 to -7), and cultured together with X-ray irradiated splenocytes of normal mice and a specific antigen (Df) or a nonspecific antigen (ME06), and the antigen-specific proliferative responses were analyzed by Cell proliferation reagent (manufactured by Roche Diagnostics). As a result, the antigen-specific proliferative responses were observed in the F1 mice Df#1-1, Df#1-3, and Df#1-7 in which the rearranged alleles of both of TCRα chains and TCRβ chains were handed over from the parent mice (see, FIG. 5). It was clarified from this matter that antigen reactivities in the cloned mice generated from antigen-specific CD4-positive T cells require both of the rearranged TCRα chains and TCRβ chains, and the phenotype would be handed over to the offspring.

### Example 6 (Antigen Reactivities 2 in CD4-Positive T Cells of Cloned Mice)

Antigen reactivities were analyzed for OVA-specific CD4-positive T cell cloned mice (OVA#6) obtained by referring to Examples 1 and 2, and Dp-specific CD4-positive T cell cloned mice obtained in Example 2 (Dp#7 to #11).

Concretely, peripheral white blood cells of cloned mice generated using OVA 326-339 partial peptide as an antigen were cultured together with OVA 326-339 partial peptide, OVA 323-339 partial peptide, or OVA protein and X-ray irradiated splenocytes of normal BALB/c mice as antigen-presenting cells (APC).

In addition, as to the cloned mice (Dp#7 to #11), their peripheral white blood cells were cultured together with Der p1 and APC.

On the fourth to fifth day from the beginning of culture, the cell proliferative responses were analyzed using a Cell proliferation reagent (manufactured by Roche Diagnostics). The results of the cloned mice (OVA#6) are shown in FIG. 6, and the results of the cloned mice (Dp#7 to #11) are shown in FIG. 7.

From FIGs. 6 and 7, all of the cells derived from cloned mice responded to the specific antigen and showed proliferative responses. It was clarified from this matter that a series of cloned mice that possess antigen specificities of a donor cell and show different antigen reactivities could be established, in the same manner as the cloned mice (Df).

### Example 7 (TCR Expression of Cloned Mice)

Whether or not the rearranged TCR chains were expressed on the surface of the cloned mouse T cells was studied. Here, of the generated cloned mice, it was previously confirmed by PCR cloning that Vβ13-1 was contained in the rearranged TCRβ chains of OVA#6 and Dp#8.

Expression of TCR Vβ13-1 in splenic CD4-positive T cells and CD8-positive T cells was confirmed by flow cytometry for cloned mice Df#1, OVA#6, Dp#7, and Dp#8. The results are shown in FIG. 8.

As a result, it was confirmed that TCR Vβ13-1 was expressed on the surface of CD4-positive T cells and CD8-positive T cells in the cloned mice of OVA#6 and Dp#8. It was clarified that the rearranged TCR chains in the cloned mice were certainly functioning by being expressed on the surface of T cells.

### Example 8 (Antigen Specificities in CD4-Positive T Cells of Cloned Mice)

Antigen specificities were studied using cloned mice Df#1, Df#2, and Dp#7.

Concretely, after CD4-positive T cells were prepared from each of the cloned mice, those cells were cultured together with APC, using a Der f crude extract (Der f), a Der f mite body extract (Der f-b), a Der p crude extract (Der p) and a Der p mite body extract (Der p-b) as antigens for Df#1 and Df#2, or on the other hand using a recombinant Der p1-GST fusion protein generated using *Escherichia coli* or various partial peptides thereof (shown in FIG. 10) as an antigen for Dp#7. On the fourth to fifth day from the beginning of culture, the cell proliferative responses were analyzed using a Cell proliferation reagent (manufactured by Roche Diagnostics). The results of the cloned mice (Df#1 and Df#2) are shown in FIG. 9, and the results of the cloned mice (Dp#7) are shown in FIG. 10.

As a result, although the CD4-positive T cells of Df#1 and Df#2 were responsive with the Der f crude extract (Der f) and Der f mite body extract (Der f-b) and showed proliferative responses, the cells were not responsive with the Der p crude extract (Der p) and the Der p mite body extract (Der p-b) (FIG. 9). On the other hand, the CD4-positive T cells of Dp#7 were responsive with a full-length mature Der p1 protein (amino acids 99-320) and a partial protein thereof (amino acids 169-248) and showed proliferative responses (FIG. 10, left diagram). Further, as a result of studies using synthetic peptides in which the partial protein was further fragmented, it was found that an epitope with which the CD4-positive T cells of Dp#7 were responsive was a partial peptide of amino acids 189-208 (FIG. 10, right diagram). It was clarified that the CD4-positive T cells of each of cloned mice showed antigen-specific reactivities.

### Example 9 (Differentiation Abilities of CD4-Positive T Cells of Cloned Mice)

The CD4-positive T cells have been known to differentiate to various Th cells in addition to being responsive to TCR stimulations to proliferate. In view of the above, the differentiation abilities of CD4-positive T cells in cloned mice which were established from Der f-specific CD4-positive T cells were studied.

It has been found that the offspring obtained by mating Df# 1 mice with normal mice have either each of rearranged Vβ2 and/or Vα6D-4 on a single-sided allele, or wild type Vβ(WT-Vβ) and Vα(WT-Vα) genes on both the alleles (see, Example 5). Similarly, it has been found that the offspring of Df#2 mice could express Vβ5 and/or Vα10. After those offspring were confirmed by genotyping, the splenic CD4-positive T cells were prepared and cultured together with anti-CD3 + anti-CD28 antibody beads or Der f antigen + APC. From the second day from the beginning of culture, IL-2 was added to promote the proliferation of responsive T cells, and collected five to ten days thereafter. The collected cells were cultured under restimulation with 1 µg/mL ionomycin + 10 nM PMA for 6 hours, and thereafter subjected to intracellular staining with an anti-IFN-γ antibody, an anti-IL-4 antibody, an anti-IL-17A antibody, and an anti-Foxp3 antibody, and expression of each of cytokines and transcription factors was analyzed by flow cytometry. The results are shown in FIG. 11.

As a result, under the stimulation conditions by the anti-CD3 + anti-CD28 antibody beads, the production of IFN-γ and IL-17A was enhanced, and the production of IL-4 was attenuated in the Df#1 mice and the Df#2 mice expressing rearranged TCR α and β, as compared to the wild-type mice of which both alleles were WT-Vβ and WT-Vα (upper row diagrams for each of mice). It was suggested that the differentiation of Th1 and Th17 was increased in these mice. Similar tendencies were also found in the Df#1 mice expressing rearranged TCRβ (the results not shown). On the other hand, in the CD4-positive T cells of the Df#1 mice and the Df#2 mice obtained by stimulations with Der f, the production of not only IFN-γ and IL-17A but also IL-4 was found by re-stimulations, whereby suggesting a possibility of being capable of differentiating to various T cell subsets such as Th1, Th2 and Th17 in live bodies more susceptible of receiving physiological antigen stimulations. In addition, the expression level of Foxp3, a differentiating marker for the regulatory T cell(Treg), was not clearly affected by expression of the rearranged TCR (bottom raw diagrams for each of mice).

### Example 10 (Bronchial Asthma-Like Airway Inflammatory Reactions in Cloned Mice)

The offspring of Df#1 mice having the rearranged Vβ2 or Vα6D-4, or wild-type Vβ(WT-Vβ) or Vα(WT-Vα) alleles were intranasally challenged with a Der f antigen once a day with an interval of 3 to 4 days for a total of from 4 to 12 times, to study the development of the bronchial asthma-like airway inflammation.

Concretely, Df#1 cloned mice, CD2F1 mice, and BALB/c mice were challenged with Der f as mentioned above and the saline (control). After 72 hours from a final challenge, tracheal cannulation was performed under anesthesia, and the respiratory system resistance (Rrs) induced by inhalation of a methacholine (MCh) solution gradually from a low concentration under the artificial respiration was measured, and expression of bronchial hyperresponsiveness (BHR), a characteristic pathophysiological change observed in bronchial asthma, was evaluated. Immediately thereafter, the mice were subjected to bronchoalveolar lavage (BAL), and the number of inflammatory cells collected in the BAL fluid (BALF) was counted. The results are shown in FIG. 12.

As a result, in the wild-type mice having WT-Vβ and WT-Vα alleles, slight increases in the number of eosinophils in the airway and the number of lymphocytes infiltrated were seen by four antigen challenges, as compared to that of the saline challenge, and the responsiveness and the number of neutrophils infiltrated were remarkably increased in the mice expressing Vβ2 and Vα6D-4 (FIG. 12, upper row). Further, even in mice expressing either one of Vβ2 or Vα6D-4, similar responsive increases were seen though they were not as much as mice expressing both. On the other hand, CD2F1 mice used as a nuclear transfer donor and BALB/c mice used in back-crossing the born cloned mice were similarly studied, and a result, slight increases in the number of eosinophils were finally seen in the CD2F1 mice by challenging with the antigen 12 times, but the responsiveness was clearly weaker than the cloned mice expressing rearranged TCR (FIG. 12, middle row).

In addition, in the Vβ2 and/or Vα6D-4 expressing mice which were challenged with an antigen four times, Rrs was increased, in other words, an airway arctation reaction was increased, by gradual inhalation of MCh, as compared to the wild-type mice having WT-Vβ and WT-Vα alleles and the control mice challenged with the saline, so that the development of bronchial-asthma-like BHR took place (FIG. 12, lower left). On the other hand, CD2F1 mice and BALB/c mice were similarly studied. As a result, a slight BHR was seen in the CD2F 1 mice finally by challenging with the antigen for 12 times, but the responsiveness was clearly weaker than that of the cloned mice expressing rearranged TCR (FIG. 12, lower left). In the cloned mice, the allergic airway inflammatory responses caused by the antigen challenges were enhanced, so that it was clarified that a bronchial asthma-like pathological model could be established in a very short time period as compared to that of the wild-type mice.

Example 11 (Allergic Rhinitis-Like Reactions in Cloned Mice) The offspring of OVA#6 mice having the rearranged Vβ13-1 or Vα4-4, or wild-type Vβ(WT-Vβ) or Vα(WT-Vα) alleles were nasally challenged with OVA once a day for five continuous days, to study the allergic rhinitis-like sneezing reactions or nasal inflammation reactions.

Concretely, OVA#6 mice and BALB/c mice were challenged with OVA and the saline (control) as mentioned above, and at each time point of the third day and the fifth day, the number of sneezes immediately after the antigen challenge (per five minutes) and the number of sneezes immediately after administration with histamine or BSA as a nonspecific stimulation 6 hours after the challenge (per five minutes) were counted. In addition, after 6 hours from a fifth antigen challenge, the mice were subjected to nasal lavage (NAL), and the number of inflammatory cells in the NAL fluid (NALF) was counted. The results are shown in FIG. 13.

As a result, in the wild-type mice having WT-Vβ and WT-Vα alleles, sneezing reactions were hardly induced even by any of third and fifth antigen challenges. On the other hand, in the mice expressing Vβ13-1 and Vα4-4, the number of sneezes immediately after the challenge was clearly larger, so that the development of a so-called immediate nasal response (INR) was found (FIG. 13, upper left). In addition, although the sneezing reactions induced by a nonspecific stimulation after the antigen challenge were hardly induced in the wild-type mice, the sneezing reactions were clearly induced in the mice expressing both Vβ13-1 and Vα4-4, and the level thereof increased together with the number of antigen challenges. Therefore, it was clarified that the development of nasal hyperresponsiveness (NHR) took place by a slight antigen challenge (FIG. 13, upper row, center and right).

As to the inflammatory reactions in nasal cavity, the mice expressing both Vβ13-1 and Vα4-4 had increased infiltrations of eosinophils, neutrophils, and lymphocytes to nasal mucosa, as compared to those of the wild-type mice (FIG. 13, bottom row). It was clarified that if the cloned mice were used, allergic rhinitis-like pathologies accompanying inflammatory cell filtrations and NHR to nasal mucosa can be established in a very short time.

### (Discussion)

By using cloned mice which was generated from CD4-positive T cells of antigen-sensitized mice of the present invention, experimental models mimicking the pathologies of bronchial asthma and allergic rhinitis can be established in a short time. Those experimental models can be widely used in various studies in future.

Concretely, it was clarified that, for example, not only Df#1 and Df#2 cloned mice but also cloned mice generated from Der p1 and OVA326-339-reactive CD4-positive T cells showed responsiveness to their specific antigens. Since at least a part of those cloned mince showed antigen specificities, it was clarified that the antigen reactivities of the donor T cell were handed over to many of the cloned mice. Further, since it could be confirmed that the rearranged TCR was expressed on the surface of the cloned mouse T cells, it was shown that the method for generating cloned mice using an antigen-specific CD4-positive T cell of the present invention is useful in obtaining a mice lineage showing an intended antigen reactivity.

In addition, although the cloned mice expressing the rearranged TCRα and TCRβ showed the tendencies of differentiating to Th1 and Th17, as compared to the wild-type mice, it was clarified that Th2 cytokine producing ability was also acquired under more physiologically stimulating conditions. It has been elucidated in the recent years that various allergic diseases show pathologies in which many subsets including Th1 and Th17 are involved in a complicated manner, not just involving a simple Th2 type disease (Kaminuma, Osamu et al., Allergy Immunity 22:1526-1534, 2015.). It is considered that the presently obtained cloned mice are suitable in analyzing the complicated allergic pathologies as mentioned above.

Further, it was clarified that the cloned mice could establish bronchial asthma-like and allergic rhinitis-like pathological models in a very short time as compared to those of the wild-type mice. In general, in an animal model for an allergic disease, an animal is systemically administered with an antigen together with an adjuvant substance such as alum to allow sensitization, and the sensitized animal is subjected to antigen challenges to a target organ to develop the pathological changes. However, in the developmental mechanisms of allergic diseases in human, since the condition of systemically administering an antigen cannot be considered, the development of models in which the allergic pathology can be developed just by a local antigen exposure has been advanced in the recent year. However, in the conventional models, in order to induce a clear bronchial asthma-like pathology, it was necessary to continue antigen challenges nearly daily for one month or longer (Shimizu H et al., Inflamm Res 62:911-917, 2013, etc.). In the present study, we also confirmed that in order to induce similar pathologies in BALB/c and CD2F1, it is necessary to perform airway antigen exposure with 3- or 4- day interval for 12 or more times. On the other hand, strong airway inflammation accompanying BHR and infiltration of inflammatory cells could be caused with as little as four times of the antigen challenges to the cloned mice, so that the present mice were shown to be highly useful as the bronchial asthma model animals. Here, in the present experimental system, in addition to infiltration of eosinophils, which are said to play a key role in the allergic diseases, infiltrations of many neutrophils and lymphocytes were found. As a result of analyzing the bronchial asthma models using antigen-specific Th cell-transferred mice which was previously established *in vitro,* it was clarified that Th2 cells especially strongly induces infiltration of eosinophils, and that Th1 and Th17 especially strongly induce infiltration of neutrophils (Kaminuma O et al., Clin Exp Allergy 42:315-325, 2012). Therefore, in the present models, the development of complex airway inflammation involving not only Th2 but also Th1 or Th17 takes place, and that is considered to reflect the differentiation tendencies of the CD4-positive T cells of the present cloned mice *in vitro.*

In addition, it was clarified that both the rearranged TCRα and TCRβ are necessary in the antigen reactivities of the CD4-positive cells in vitro in the cloned mice; however, it was clarified that the extent of the allergic inflammation to be developed *in vivo* is enhanced even in mice expressing only one of the rearranged TCRα or TCRβ, as compared to that of the wild-type mice. Although the causations are unclear, when CD4-positive T cells expressing a single rearranged TCR receive antigen presentation plural times *in vivo,* it is considered to be possible to form an antigen-reactive TCR heterodimer with a higher probability than T cells in which both are from the wild-type alleles. Even in human, a possibility that a certain kind of TCR repertoire is involved in the development of an allergic disease has been shown (Bankakos P et al., Clin Exp Immunol 128:295-301, 2002). It is considered that the state of lowering a threshold of the antigen reactivity by expressing a single rearranged TCR reflects the role of the single TCR repertoire as the causations of the allergy mentioned above, which is said to further show the high usefulness of the present cloned mice.

Surprisingly, when the cloned mice were used in allergic rhinitis models, the development of the nasal inflammation accompanying INR, NHR, and nasal inflammation infiltration was confirmed in as short as 3 to 5 days. It was shown that the present cloned mice are useful in the analysis of pathologies of not only bronchial asthma but also allergic rhinitis. We have already clarified that the antigen-provoked NHR and inflammatory cell infiltration reactions in the sensitized mice are controlled primarily by CD4-positive T cells (Nishimura T et al., PLOS ONE, under revision), and it is said that the present results are consistent with the above. Further, by using the present model in a detailed analysis, the cloned mice are expected to be useful in the elucidation of the pathologies of allergic rhinitis.

In this study, the bronchial asthma and allergic rhinitis models which develop in a short time were successfully generated by utilizing antigen-specific CD4-positive T cell cloned mice, whereby the usefulness of the present cloned mice in the pathological analysis or pharmacological efficacy evaluations of the medicaments was clarified. In future, it is suggested that the antigen-specific CD4-positive T cells-derived cloned mice can greatly contribute to studies of various diseases by pursuing possibilities of applying the cloned mice to other allergic or non-allergic diseases.

### INDUSTRIAL APPLICABILITY

Since the non-human mammal of the present invention shows allergic reactions specific to a mite antigen surely and efficiently, the non-human mammal can be suitably applied as a developmental model of allergic diseases.

### SEQUENCE LISTING FREE TEXT

SEQ ID NO: 1 of the Sequence Listing is a partial peptide consisting of the amino acid sequence 169 to 188 of *Dermatophagoides farinae* protein.
SEQ ID NO: 2 of the Sequence Listing is a partial peptide consisting of the amino acid sequence 179 to 198 of *Dermatophagoides farinae* protein.
SEQ ID NO: 3 of the Sequence Listing is a partial peptide consisting of the amino acid sequence 189 to 208 of *Dermatophagoides farinae* protein.
SEQ ID NO: 4 of the Sequence Listing is a partial peptide consisting of the amino acid sequence 202 to 221 of *Dermatophagoides farinae* protein.
SEQ ID NO: 5 of the Sequence Listing is a partial peptide consisting of the amino acid sequence 209 to 228 of *Dermatophagoides farinae* protein.
SEQ ID NO: 6 of the Sequence Listing is a partial peptide consisting of the amino acid sequence 219 to 238 of *Dermatophagoides farinae* protein.
SEQ ID NO: 7 of the Sequence Listing is a partial peptide consisting of the amino acid sequence 229 to 248 of *Dermatophagoides farinae* protein.

## Claims

1. A non-human mammal and an offspring thereof, obtainable by a somatic cell nuclear transfer method using a nucleus of a CD4-positive T cell as a nuclear donor.

2. The non-human mammal and an offspring thereof according to claim 1, wherein the CD4-positive T cell is a CD4-positive T cell at G₀ phase and/or G₁ phase.

3. The non-human mammal and an offspring thereof according to claim 1 or 2, wherein a gene region for a T cell receptor is rearranged as a T cell receptor specific to a mite antigen.

4. The non-human mammal and an offspring thereof according to claim 3, wherein the mite antigen is a mite body or excrement of *Dermatophagoides farinae or Dermatophagoides pteronyssinus*, or an extract, a recombinant protein or a synthetic peptide thereof.

5. The non-human mammal and an offspring thereof according to claim 1 or 2, wherein a gene region for a T cell receptor is rearranged as a T cell receptor specific to an egg albumin.

6. A method for preparing a non-human mammal comprising performing a somatic cell nuclear transfer using a nucleus of a CD4-positive T cell as a nuclear donor.

7. The method according to claim 6, wherein the CD4-positive T cell is a CD4-positive T cell obtainable by adding IL-2 thereto, culturing the mixture, thereafter removing IL-2, and further culturing the cultured mixture.

8. The method according to claim 6 or 7, wherein a gene region for a T cell receptor is rearranged as a T cell receptor specific to a mite antigen.

9. The method according to claim 8, wherein the mite antigen is a mite body or excrement of *Dermatophagoides farinae or Dermatophagoides pteronyssinus*, or an extract, a recombinant protein or a synthetic peptide thereof.

10. The method according to claim 6 or 7, wherein a gene region for a T cell receptor is rearranged as a T cell receptor specific to an egg albumin.

11. A non-human mammal wherein a gene region for a T cell receptor is rearranged as a T cell receptor specific to a mite antigen or an egg albumin, and wherein a single T cell receptor derived from the gene region is expressed in an individual.

12. The non-human mammal according to claim 11, wherein the mite antigen is a mite body or excrement of *Dermatophagoides farinae or Dermatophagoides pteronyssinus*, or an extract, a recombinant protein or a synthetic peptide thereof.
